# FASCICULE DE BREVET EUROPEEN

(11) **EP 0 927 882 B1**
(45) Date de publication et mention de la délivrance du brevet: **24.10.2001**
(21) Numéro de dépôt: 98403319.1
(22) Date de dépôt: 29.12.1998
(51) Int. Cl.: G01N 21/77, G02B 6/24

(54) **Dispositif optique de détection in situ de traces d'hydrogène gazeux dans un environnement à températures cryogéniques**
Optische Vorrichtung zur in-situ-Detektion von Spuren gasförmigen Wasserstoffs in einer Tieftemperaturumgebung
Optical apparatus for in situ detection of traces of hydrogen gas in a cryogenic temperature environment

(30) Priorité: 29.12.1997 FR 9716614
(43) Date de publication de la demande: 07.07.1999
(73) Titulaire: SOCIETE NATIONALE D'ETUDE ET DE CONSTRUCTION DE MOTEURS D'AVIATION, "S.N.E.C.M.A.", 75015 Paris (FR)
(72) Inventeur: Bevenot, Xavier, 42000 Saint-Etienne (FR); Clement, Michel, 27200 Vernon (FR); Gagnaire, Henri, 42400 St.Chamond (FR)
(74) Mandataire: Thévenet, Jean-Bruno

(56) Documents cités:
- BUTLER M A: "MICROMIRROR OPTICAL-FIBER HYDROGEN SENSOR" SENSORS AND ACTUATORS B, vol. B22, no. 2, 1 novembre 1994, pages 155-163, XP000486125
- WANG ET AL: "RESEARCH OF FIBER-OPTIC HYDROGEN SENSOR" PROCEEDINGS OF THE SPIE: SECOND INTERNATIONAL SYMPOSIUM ON MEASUREMENT TECHNOLOGY AND INTELLIGENT INSTRUMENTS, WUHAN, CHINA, 29 OCTOBER - 5 NOVEMBER 1993, vol. 2101, no. 2, 1993, pages 1139-1141, XP002076889

## Description

### Domaine de l'invention

La présente invention a pour objet un dispositif optique de détection in situ de traces d'hydrogène gazeux dans un environnement à températures cryogéniques, à partir d'un capteur comprenant un film fin de palladium dont les propriétés optiques varient en fonction de la concentration d'hydrogène en contact avec le film.

### Art antérieur

On connaît déjà l'utilisation de capteurs d'hydrogène à micromiroir conçus pour détecter l'hydrogène près de la limite explosive, pour une concentration de l'ordre de 4 % dans l'air, comme décrit par exemple dans: Butler MA: "Micromirror optical-fiber hydrogen sensor", Sensors and Actuators, vol. B22 (1994), no. 2, pages 155-163.

De tels capteurs comprennent un support tel qu'une fibre optique comportant une gaine et un coeur et sur laquelle il est déposé en bout de fibre, par évaporation, un film de palladium. En présence d'hydrogène, le film se transforme en un hydride de la forme Pd Hₓ. Une modification de la concentration d'hydrogène a pour effet une modification de la composition de l'hydride (variation de x) mais également de sa structure électronique, ce qui engendre une variation de la réflectivité du film. La mesure de la lumière réfléchie permet alors de déduire la concentration d'hydrogène.

L'utilisation de tels capteurs a été préconisée pour des températures ambiantes ou des températures supérieures à la température ambiante.

En revanche, la mise en oeuvre de tels capteurs dans des milieux à températures cryogéniques ne permettent pas, dans les conditions opératoires traditionnelles, d'obtenir une sensibilité satisfaisante ou un temps de réponse suffisamment court.

### Objet et description succincte de l'invention

La présente invention vise à remédier aux inconvénients précités et à permettre de détecter de façon fiable, avec un temps de réponse court et une sensibilité satisfaisante, à l'aide d'un dispositif peu encombrant placé in situ, la présence de traces d'hydrogène gazeux dans un environnement à températures cryogéniques, par exemple de l'ordre de 20 à 80 K.

Ces buts sont atteints, conformément à l'invention, grâce à un dispositif optique de détection in situ de traces d'hydrogène gazeux dans un environnement à températures cryogéniques, à partir d'un capteur comprenant un film fin de palladium dont les propriétés optiques varient en fonction de la concentration d'hydrogène en contact avec le film, caractérisé en ce qu'il comprend un micromiroir de palladium déposé sous forme de film à une première extrémité d'une première fibre optique, une première source lumineuse émettant un signal lumineux de longueur d'onde prédéterminée à une deuxième extrémité de la première fibre optique, des moyens de détection du signal lumineux modifié après passage au contact du micromiroir de palladium disposé dans l'environnement à températures cryogéniques, des circuits de traitement pour déterminer, à partir de la variation d'intensité du signal lumineux au contact du micromiroir de palladium, la concentration en hydrogène, et des moyens de chauffage localisé du micromiroir de palladium par rayonnement lumineux de manière à maintenir le micromiroir dans la phase α du palladium.

Selon un premier mode de réalisation, le dispositif comprend une première source lumineuse constituée par une diode laser de longueur d'onde λ₁ située dans le proche infrarouge et de puissance inférieure à quelques dizaines de milliwatts pour émettre à travers un coupleur en Y un signal de détection à ladite deuxième extrémité de la première fibre optique pour éclairer la face arrière du micromiroir de palladium , le coupleur en Y recevant en retour le signal lumineux de longueur d'onde λ₁ réfléchi par le micromiroir de palladium et appliquant ce signal lumineux réfléchi à travers un filtre interférentiel auxdits moyens de détection , et les moyens de chauffage localisé du micromiroir de palladium par rayonnement lumineux comprennent une deuxième source lumineuse constituée par une diode laser de longueur d'onde λ₂ située dans l'infrarouge, supérieure à la longueur d'onde λ₁ et émettant de façon continue un faisceau de puissance comprise entre quelques dizaines et quelques centaines de milliwatts, à une première extrémité d'une deuxième fibre optique dont une deuxième extrémité est située à faible distance du micromiroir de palladium dans l'environnement à températures cryogéniques de manière à assurer un chauffage de la face avant du micromiroir par le rayonnement lumineux issu de la deuxième source lumineuse et de la fibre optique .

De façon plus particulière, la première extrémité de la première fibre optique et la deuxième extrémité de la deuxième fibre optique sont réunies par une férule en matériau transparent munie d'orifices de communication avec l'environnement à températures cryogéniques.

Selon un deuxième mode de réalisation, le dispositif comprend une première source lumineuse constituée par une diode laser de longueur d'onde λ située dans l'infrarouge et de puissance comprise entre quelques dizaines de milliwatts et quelques centaines de milliwatts pour émettre de façon continue à travers un coupleur en Y un signal de détection à ladite deuxième extrémité de la première fibre optique pour éclairer la face arrière du micromiroir de palladium en assurant un chauffage localisé de cette face arrière, le coupleur en Y recevant en retour le signal lumineux de longueur d'onde λ réfléchi par le micromiroir de palladium et appliquant ce signal lumineux réfléchi auxdits moyens de détection.

Selon un troisième mode de réalisation, le dispositif comprend une première source lumineuse constituée par une diode laser de longueur d'onde λ située dans l'infrarouge et de puissance comprise entre quelques dizaines de milliwatts et quelques centaines de milliwatts pour émettre de façon continue à travers un isolateur optique un signal de détection à ladite deuxième extrémité de la première fibre optique pour éclairer la face arrière du micromiroir de palladium situé à la première extrémité de la première fibre optique en assurant un chauffage localisé de cette face arrière, le signal lumineux de longueur d'onde λ étant transmis à travers le micromiroir de palladium semi-transparent à une première extrémité d'une deuxième fibre optique mise en vis-à-vis de la deuxième extrémité de la première fibre optique portant le micromiroir de palladium , la deuxième fibre optique présentant un diamètre de coeur équivalent ou supérieur à celui de la première fibre optique et appliquant par sa deuxième extrémité le signal lumineux transmis à travers le micromiroir de palladium auxdits moyens de détection.

Dans ce cas, avantageusement, la première fibre optique est fixée par collage au voisinage de sa première extrémité à un support transparent et muni d'orifices de communication avec l'environnement à températures cryogéniques et la deuxième fibre optique est également fixée par collage, au voisinage de sa première extrémité, au support de telle manière que la distance entre la première extrémité de la première fibre et la première extrémité de la deuxième fibre soit de l'ordre de quelques dizaines de micromètres.

L'environnement à températures cryogéniques peut être constitué par une atmosphère gazeuse, telle que de l'azote ou par un fluide liquide tel qu'une huile de refroidissement.

Le dispositif selon l'invention peut être appliqué notamment dans le domaine spatial, par exemple à la détection de fuites d'hydrogène à l'intérieur du calorifugeage de lignes de transport d'ergol dans un lanceur.

### Brève description des dessins

D'autres caractéristiques et avantages de l'invention ressortiront de la description suivante de modes particuliers de réalisation, donnés à titre d'exemples, en référence aux dessins annexés, sur lesquels :
- la figure 1 est une vue schématique d'un exemple de dispositif optique de détection d'hydrogène gazeux, selon un premier mode de réalisation de l'invention, avec deux sources de lumière,
- la figure 2 est une vue schématique d'un exemple de disposition optique de détection d'hydrogène gazeux selon un deuxième mode de réalisation de l'invention utilisant une seule source de lumière, et
- la figure 3 est une vue schématique d'un exemple de disposition optique de détection d'hydrogène gazeux selon un troisième mode de réalisation de l'invention utilisant une seule source de lumière.

### Description détaillée de modes particuliers de réalisation

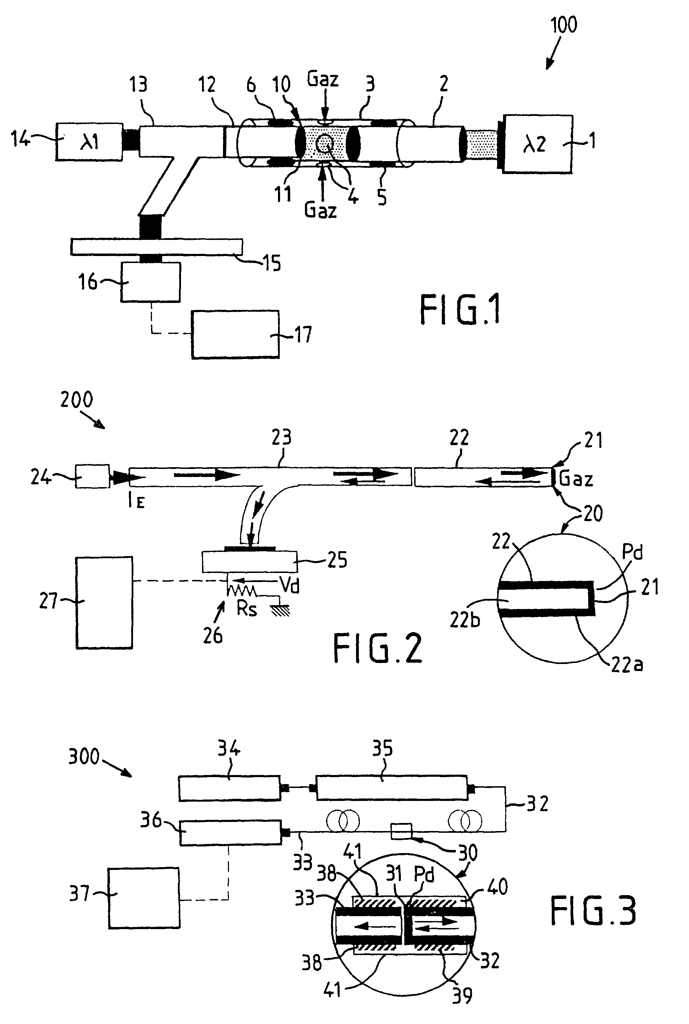

On considèrera en premier lieu le mode de réalisation de la figure 1, sur lequel on voit une première fibre optique 12 dont une extrémité est recouverte d'un film métallique de palladium constituant un micromiroir semi-transparent. Ce micromiroir de palladium 11 constitue l'élément actif du capteur 10 en contact avec un milieu environnant dans lequel de l'hydrogène gazeux est susceptible d'être présent et doit pouvoir être détecté, même à l'état de traces.

La fibre optique 12 à la première extrémité de laquelle est déposé le micromiroir de palladium 11 peut présenter par exemple un diamètre de coeur de l'ordre de 50 micromètres et un diamètre de gaine de l'ordre de 125 micromètres.

Le micromiroir de palladium 11 peut être constitué par un film dont l'épaisseur est comprise entre 10 et 50 nanomètres et de préférence comprise entre 15 et 30 nanomètres.

La deuxième extrémité de la fibre optique 12 est elle-même connectée à un coupleur en Y 13 dont la branche principale reçoit un faisceau lumineux d'une diode laser 14 dont la puissance est inférieure à quelques dizaines de milliwatts et qui émet un signal lumineux de longueur d'onde λ₁ située dans le proche infrarouge. La branche en dérivation du coupleur 13 qui reçoit de la fibre optique 12 la lumière rétroréfléchie par le micromiroir 11 dirige cette lumière réfléchie vers un détecteur 16 à travers un filtre interférentiel 15 dont le rôle sera explicité plus loin.

Le module d'émission d'un faisceau laser comprenant la diode laser 14 peut être déporté hors de la zone de mesure située dans un environnement à températures cryogéniques, à l'aide d'une fibre optique connectée à la branche principale du coupleur 13.

De la même façon, le filtre interférentiel 15, ainsi que le détecteur 16 et des circuits 17 de traitement des signaux issus du détecteur 16 peuvent être déportés hors de la zone de mesure située dans un environnement à températures cryogéniques, à l'aide d'une fibre optique connectée à la branche en dérivation du coupleur 13 recevant le signal lumineux réfléchi par le micromiroir 11.

Le détecteur 16, qui peut être constitué par un radiomètre ou tout autre mesureur de puissance lumineuse adapté à la puissance de la diode laser 14, fournit en temps réel aux circuits de traitement 17, qui peuvent comprendre un système d'acquisition numérique ou analogique, des informations concernant la puissance lumineuse réfléchie par le micromiroir 11. La mesure de la lumière réfléchie permet de déduire la concentration d'hydrogène dans le milieu avec lequel est en contact le micromiroir 11, dès lors que la présence d'hydrogène au contact du micromiroir 11 crée un hydride Pd Hₓ dont la composition est fonction de la concentration en hydrogène et détermine une réflectivité qui varie de façon déterminée, réversible et reproductible en fonction de la concentration en hydrogène.

D'une manière générale, plus la concentration d'hydrogène est élevée, plus la réponse du capteur à micromiroir de palladium 11 est rapide et marquée. Par ailleurs, les variations de la réflectivité fractionnelle d'un film de Pd H_{X} en fonction de la concentration en hydrogène révèlent que l'opacité du film présente une augmentation rapide jusqu'à 2 % de concentration en hydrogène , puis une augmentation plus lente pour une concentration en hydrogène allant de 2 à 100 %. La linéarité de la courbe donnant les variations de la réflectivité fractionnelle en fonction de la concentration en hydrogène présente toutefois une linéarité parfaite entre 2 et 100 % de concentration en hydrogène. Le système de détection selon l'invention permet ainsi non seulement une détection mais également, après étalonnage du système, une évaluation de la concentration en hydrogène, à partir de la mesure de la quantité de lumière rétroréfléchie par le micromiroir 11.

Dans le cas de détection de traces d'hydrogène, notamment lorsqu'il convient de détecter ou évaluer des concentrations en hydrogène inférieures à la concentration limite (de l'ordre de 4 %) à partir de laquelle un mélange gazeux contenant de l'hydrogène devient explosif, il s'avère qu'un capteur à micromiroir de palladium s'avère satisfaisant à la température ambiante ou à une température plus élevée que la température ambiante.

En revanche, le temps de réponse s'avère beaucoup trop long (de l'ordre de plusieurs heures) et donc inadapté à de nombreuses applications lorsque le micromiroir de palladium 11 se trouve situé dans un milieu à températures cryogéniques, comme par exemple dans les moteurs-fusées où, pour des questions de sécurité, il est souhaitable de pouvoir détecter des fuites d'hydrogène avec un temps de réaction très court, de l'ordre de quelques secondes, par exemple au niveau de l'espace entre une conduite d'ergol et le calorifugeage de cette conduite.

Pour remédier à cet inconvénient, et obtenir des temps de réponse courts, conformément à l'invention, on procède à un chauffage localisé du micromiroir de palladium 11 de manière à maintenir le micromiroir dans la phase α du système palladium-hydrogène.

Le système palladium-hydrogène Pd Hₓ possède deux phases α et β, avec un domaine assez large où ces deux phases coexistent :
- lorsque x < 0,03, seule la phase α existe,
- lorsque 0,03 < x < 0,6 les deux phases α et β coexistent,
- lorsque x > 0,6, seule la phase β existe.

A très basse température, par exemple à - 196°C, le système palladium-hydrogène, lorsque le palladium est mis en contact avec de l'hydrogène dans une faible concentration, par exemple de l'ordre de 4 %, dans un milieu tel que l'azote, se trouve dans sa phase β et le temps de réaction du capteur est prohibitif, de l'ordre de plusieurs heures.

Selon l'invention, en procédant à un chauffage localisé suffisant du micromiroir de palladium 11 pour amener le système palladium-hydrogène dans sa phase α, le temps de réaction est ramené à quelques secondes, avec une sensibilité satisfaisante.

Selon le mode de réalisation de la Figure 1, le chauffage localisé du micromiroir 11 est réalisé à l'aide d'un faisceau de lumière cohérente appliqué sur la face avant du micromiroir 11. Pour cela, on utilise une diode laser 1 de puissance émettant un faisceau lumineux de longueur d'onde λ₂ située dans l'infrarouge, et de préférence supérieure à la longueur d'onde λ₁ du faisceau lumineux émis par la diode laser 14. Avantageusement, la diode laser 1 émet de façon continue un faisceau de puissance comprise entre quelques dizaines et quelques centaines de milliwatts.

La diode laser 1 émet un faisceau lumineux à une première extrémité d'une fibre optique 2 dont une deuxième extrémité est située à faible distance du micromiroir de palladium 11 dans l'environnement à températures cryogéniques. La fibre optique 2 peut être suffisamment longue, ou être composée d'une série de fibres optiques connectées entre elles, de telle sorte que la diode laser 1 peut le cas échéant se situer elle-même en dehors de la zone de mesure comprenant un milieu ambiant à températures cryogéniques.

Comme on peut le voir sur la figure 1, la première fibre optique 12 peut être rendue solidaire d'une férule 3 en verre, par exemple par une liaison 6 par collage, dans des zones voisines de sa première extrémité portant le micromiroir 11 tandis que la fibre optique 2 peut être rendue solidaire de cette même férule 3, par exemple par une liaison 5 par collage, dans des zones voisines de sa deuxième extrémité située à proximité du micromiroir 11. La férule en verre 3, qui comporte des orifices 4 de communication avec l'environnement à températures cryogéniques susceptible de contenir de l'hydrogène, assure un maintien en position face à face de la deuxième extrémité de la fibre optique 2 et de l'extrémité de la fibre optique 12 recouverte du micromiroir 11 de telle sorte que le faisceau lumineux de puissance issu de la fibre optique 2 frappe directement la face avant du micromiroir 11.

Une partie du rayonnement émis par la fibre optique 2 à partir de la diode de puissance 1 de longueur d'onde λ₂ traverse le micromiroir 11. Le filtre interférentiel 15 empêche que ce rayonnement résiduel de longueur d'onde λ₂ arrive sur le détecteur 16 et garantit que le détecteur 16 ne prend en compte que le rayonnement de longueur d'onde λ₁ émis par la diode laser 14 et rétroréfléchi par le micromiroir 11.

Dans le dispositif 100 selon l'invention illustré sur la figure 1, grâce à l'absorption locale par le micromiroir 11 de l'énergie lumineuse fournie par la diode laser 1, la température du métal du micromiroir 11 est élevée localement de sorte que par exemple, on peut obtenir un temps de montée tm (passage de 10% à 90% de la valeur finale de la réponse du capteur) inférieur à trois secondes pour la détection de 4% d'hydrogène dans l'azote (présentant un débit de 500 Nl/h) avec une température du gaz de - 196°C. La température du palladium est ainsi élevée à une valeur telle que le métal se trouve dans sa phase α. La variation relative du signal (Vr) correspondant au passage d'une atmosphère de 100% d'azote à x% d'hydrogène est de quelques pour cent lorsque le palladium est dans sa phase α de sorte que la détection peut s'effectuer non seulement de façon rapide, mais également de façon fiable avec une sensibilité suffisante.

Le choix pour la diode laser 1 de chauffage d'une longueur d'onde λ₂ différente de la longueur d'onde λ₁ de la diode laser 14 émettant le signal de détection permet de discriminer les deux signaux lumineux dans le filtre interférentiel 15 placé devant le détecteur 16. Par ailleurs, la longueur d'onde λ₂ est de préférence supérieure à λ₁ car l'absorption de l'énergie dans le métal du micromiroir 11 augmente lorsque la longueur d'onde augmente.

La figure 2 montre un deuxième mode de réalisation de l'invention dans lequel le dispositif optique de détection 200 comporte une seule source lumineuse 22 qui joue le rôle à la fois des diodes laser 14 et 1 de la figure 1.

Dans ce cas, le montage de la source lumineuse 24, du coupleur en Y 23, de la fibre optique 22 et du micromiroir 21 peut s'effectuer comme celui des éléments correspondants 14, 13, 12 et 11 de la figure 1. Les éléments 24, 23, 22, 21 ne seront donc pas décrits à nouveau en détail. Toutefois, le montage est simplifié par la suppression de la deuxième source laser 1, de la deuxième fibre optique 2 et des moyens 3 à 6 de positionnement relative des extrémités des fibres optiques 12 et 2 en regard l'une de l'autre. Par ailleurs, dans la réalisation de la figure 2, il n'est pas non plus nécessaire d'utiliser un filtre interférentiel tel que le filtre 15 de la figure 1 et un récepteur-détecteur 25-26 peut être disposé directement en sortie de la branche de dérivation du coupleur 23. Des circuits de traitement du signal 27 sont par ailleurs associés à l'ensemble récepteur-détecteur 25-26.

Le micromiroir de palladium 21 est placé à l'extrémité de la fibre optique 22 qui comprend une gaine 22a et un coeur 22b (voir la loupe de la figure 2). L'extrémité de la fibre optique 22 et le micromiroir 21 constituent un capteur d'extrémité miniature 20 disposé in situ dans un système où l'on souhaite mesurer ou détecter un niveau de fuite d'hydrogène. Le capteur d'extrémité 20, comme le capteur 10, peut être placé à proximité immédiate des interfaces ou assemblages censés fuir ou être intégrés à ces derniers. Il peut aussi permettre de mesurer la concentration en hydrogène dans une enceinte, ou encore de détecter la présence de traces d'hydrogène dans un fluide liquide tel qu'une huile de refroidissement.

Comme dans le cas du dispositif 100 de la figure 1, le dispositif 200 peut comprendre des fibres optiques supplémentaires qui sont connectées d'une part entre le coupleur 23 et la source lumineuse 24 et d'autre part entre le coupleur 23 et le récepteur-détecteur 25, 26, afin de déporter hors de la zone de mesure en ambiance cryogénique la source 24 et les moyens de détection et de traitement du signal 25 à 27.

Pour que le dispositif 200 de la figure 2 puisse permettre des mesures avec un temps de montée faible et une sensibilité suffisante dans des environnements à températures cryogéniques, avec des performances équivalentes à celles du dispositif 100 de la figure 1, il est nécessaire que la source lumineuse 24 assure une double fonction d'émission d'un signal lumineux de détection et d'émission d'un faisceau lumineux de chauffage du micromiroir 21. Pour cela, il convient de choisir une source lumineuse telle qu'une diode laser émettant de façon continue un signal lumineux avec une longueur d'onde λ située dans l'infrarouge (et non le proche infrarouge) et présentant une puissance comprise entre plusieurs dizaines de milliwatts et plusieurs centaines de milliwatts de telle sorte que le système palladium-hydrogène du micromiroir 21 se trouve dans sa phase α malgré l'environnement à températures cryogéniques dans lequel est placé le capteur 20.

Dans le mode de réalisation de la figure 2, le faisceau lumineux fourni par la source 24 est à la fois le vecteur porteur de l'information issue de la détection d'hydrogène et le vecteur porteur de l'énergie à fournir au micromiroir de palladium 21 pour évaluer sa température.

Dans ce cas, le chauffage du micromiroir 21 s'effectue par la face arrière tandis que dans le mode de réalisation de la figure 1 le chauffage est effectué par la face avant du micromiroir 11. En revanche, le principe de détection reste le même pour les modes de réalisation des figures 1 et 2 et c'est le signal lumineux émis par la source 24 et rétroréfléchi par le micromiroir 21 qui, après dérivation par le coupleur en Y 23, est détecté par l'ensemble de détection 25, 26.

Sans qu'il soit nécessaire d'utiliser deux sources lumineuses de longueurs d'onde différentes, dès lors que la puissance de la diode laser 24 est surdimensionnée par rapport à celle qui serait simplement nécessaire pour assurer une détection du signal réfléchi, de telle sorte que la température du palladium est localement élevée de façon suffisante pour que le palladium soit dans sa phase α, on peut assurer avec le dispositif de mesure 200 une détection de traces d'hydrogène dans un environnement cryogénique dans les conditions mentionnées plus haut en référence au dispositif 100, à savoir par exemple détecter 4% d'hydrogène dans de l'azote à -196°C avec un temps de montée tm inférieur à 3 secondes et une variation relative du signal Vr de quelques pour cent.

La figure 3 montre un dispositif optique 300 de détection in situ de traces d'hydrogène gazeux dans un environnement à températures cryogéniques selon un troisième mode de réalisation de l'invention dans lequel il est procédé à une détection de la présence d'hydrogène par la mesure de la variation d'intensité lumineuse transmise (et non pas réfléchie) par un micromiroir de palladium 31 placé à l'extrémité d'une fibre optique 32 recevant de la lumière d'une source lumineuse 34.

L'extrémité de la fibre optique 32 munie du micromiroir 31 est mise en vis-à-vis de l'extrémité d'une deuxième fibre optique 33 dont le diamètre de coeur est équivalent ou supérieur à celui de la première fibre optique 32. La deuxième fibre optique 32 sert au transport du signal lumineux transmis à travers le micromiroir 31, vers un détecteur 36 associé à un système de traitement du signal 37.

La configuration du dispositif de la figure 3 permet de se passer de l'utilisation d'un coupleur, ce qui diminue les pertes optiques entre la source de lumière 34 et le micromiroir 31 tout en maximisant la puissance utilisée pour procéder à la détection du signal.

La puissance de la source lumineuse 34, constituée par une diode laser de longueur d'onde λ située dans l'infrarouge, doit être comprise entre quelques dizaines de milliwatts et quelques centaines de milliwatts, c'est-à-dire doit permettre, comme les diodes laser de chauffage 1 et 24 des figures 1 et 2, de provoquer localement une élévation de la température du micromiroir 31.

L'utilisation d'un capteur 30 à micromiroir de palladium 31 utilisé en transmission permet ainsi de maximiser l'efficacité de chauffage ainsi que l'intensité du signal servant à la détection.

La variation relative du signal (Vr) correspondant à la détection de 4% d'hydrogène dans l'azote à des températures cryogéniques et le temps de montée (tm) sont similaires aux valeurs obtenues dans les mêmes conditions d'utilisation pour un capteur 10 ou 20 utilisé en réflexion.

On notera que le micromiroir 31 réfléchit de façon non souhaitable une certaine puissance lumineuse vers la source 34. Pour limiter cet inconvénient, un isolateur optique 35 est interposé entre la diode laser 34 et la fibre optique 32.

La loupe de la figure 3 montre un exemple de capteur 30 dans lequel les extrémités en vis-à-vis de la fibre optique 33 et de la fibre optique 32 sur laquelle a été déposé le micromiroir de palladium 31, sont maintenues dans des positions relatives bien définies, avec un écartement de l'ordre de quelques micromètres à quelques dizaines de micromètres, grâce à un support 40 en forme de plaquette ou de férule, par exemple en verre, qui est collée en des zones 39, 38 sur les fibres optiques 32, 33 au voisinage de leurs extrémités en vis-à-vis, et présente des ouvertures 41 de communication avec l'environnement à températures cryogéniques.

Le capteur 30 est particulièrement adapté à la détection volumique de traces d'hydrogène dans une enceinte.

Comme dans le cas des modes de réalisation des figures 1 et 2, les éléments 34, 35 et 36, 37 peuvent être déportés hors du milieu à températures cryogéniques par des fibres optiques additionnelles connectées aux fibres optiques 32, 33 respectivement.

Quel que soit son mode de réalisation, le dispositif de détection selon l'invention présente un encombrement et un poids réduits, est insensible aux perturbations électromagnétiques et présente une grande sécurité d'utilisation en milieu explosif, tout en pouvant être réalisé à un coût raisonnable.

De façon plus particulière, on peut utiliser pour le chauffage du micromiroir de palladium des diodes laser de puissance dont les longueurs d'onde sont situées dans la gamme 670 nm, 1500 nm. A titre d'exemple, une diode laser de puissance dont la longueur d'onde est 810 nm donne de bons résultats.

On a indiqué plus haut qu'une puissance lumineuse de chauffage minimum doit être appliquée au micromiroir de palladium pour maintenir celui-ci dans la phase α du palladium.

Il a été établi qu'une puissance Pₘ égale à 70 mW permet au Palladium de rester dans la phase α pour une température comprise entre 23°C (ou supérieure) et -196°C avec une concentration en Hydrogène pouvant varier de 0 % à 4 % dans l'Azote.

Pour une concentration en Hydrogène variant de 4 % à 100 %, une puissance Pₘ supérieure à 50 mW permet au Palladium d'être dans la phase α à 23°C. La puissance Pₘ nécessaire pour que le Palladium soit dans la phase α à -196°C (pour des concentrations en Hydrogène variant de 4 % à 100 %) doit être supérieure à environ 350 mW.

En fonction des puissances exprimées précédemment, le choix de la puissance de la diode Laser dépend principalement des pertes optiques existantes entre la source et le micromiroir. Dans le cas de l'utilisation d'un coupleur en Y permettant une répartition de puissance symétrique (50/50), la perte totale liée à l'utilisation de ce coupleur et de la connectique pour insérer le capteur à ce coupleur est égale à 58 %. Compte-tenu de cette perte, pour ce montage particulier (choix du type de fibre et du coupleur), la puissance minimum de la source nécessaire pour un fonctionnement en phase α de 23°C à -196°C afin de détecter jusqu'à 4% d'hydrogène dans l'azote est de l'ordre de 170 mW. Pour un fonctionnement identique jusqu'à 100 % d'hydrogène il faut une source d'environ 730 mW.

## Revendications

1. Dispositif optique de détection in situ de traces d'hydrogène gazeux dans un environnement à températures cryogéniques, à partir d'un capteur comprenant un film fin de palladium dont les propriétés optiques varient en fonction de la concentration d'hydrogène en contact avec le film, le dispositif comprenant un micromiroir de palladium (11 ; 21 ; 31) déposé sous forme de film à une première extrémité d'une première fibre optique (12 ; 22 ; 32), une première source lumineuse (14 ; 24 ; 34) émettant un signal lumineux de longueur d'onde prédéterminée à une deuxième extrémité de la première fibre optique (12 ; 22 ; 32), des moyens de détection (16;26;36) du signal lumineux modifié après passage au contact du micromiroir de palladium (11 ; 21 ; 31) disposé dans l'environnement à températures cryogéniques, des circuits de traitement (17 ; 27 ; 37) pour déterminer, à partir de la variation d'intensité du signal lumineux au contact du micromiroir de palladium (11 ; 21 ; 31) la concentration en hydrogène, et des moyens de chauffage localisé du micromiroir de palladium (11 ; 21 ; 31) par rayonnement lumineux de manière à maintenir le micromiroir dans la phase α du palladium.

2. Dispositif selon la revendication 1, **caractérisé en ce qu'**il comprend une première source lumineuse (14) constituée par une diode laser de longueur d'onde λ₁ située dans le proche infrarouge et de puissance inférieure à quelques dizaines de milliwatts pour émettre à travers un coupleur en Y (13) un signal de détection à ladite deuxième extrémité de la première fibre optique (12) pour éclairer la face arrière du micromiroir de palladium (11), le coupleur en Y (13) recevant en retour le signal lumineux de longueur d'onde λ₁ réfléchi par le micromiroir de palladium (11) et appliquant ce signal lumineux réfléchi à travers un filtre interférentiel (15) auxdits moyens de détection (16), et **en ce que** les moyens de chauffage localisé du micromiroir de palladium (11) par rayonnement lumineux comprennent une deuxième source lumineuse (1) constituée par une diode laser de longueur d'onde λ₂ située dans l'infrarouge, supérieure à la longueur d'onde λ₁ et émettant de façon continue un faisceau de puissance comprise entre quelques dizaines et quelques centaines de milliwatts, à une première extrémité d'une deuxième fibre optique (2) dont une deuxième extrémité est située à faible distance du micromiroir de palladium (11) dans l'environnement à températures cryogéniques de manière à assurer un chauffage de la face avant du micromiroir (11) par le rayonnement lumineux issu de la deuxième source lumineuse (1) et de la fibre optique (2).

3. Dispositif selon la revendication 2, **caractérisé en ce que** la première extrémité de la première fibre optique (12) et la deuxième extrémité de la deuxième fibre optique (2) sont réunies par une férule (3) en matériau transparent munie d'orifices (4) de communication avec l'environnement à températures cryogéniques.

4. Dispositif selon la revendication 1, **caractérisé en ce qu'**il comprend une première source lumineuse (24) constituée par une diode laser de longueur d'onde λ située dans l'infrarouge et de puissance comprise entre quelques dizaines de milliwatts et quelques centaines de milliwatts pour émettre de façon continue à travers un coupleur en Y (23) un signal de détection à ladite deuxième extrémité de la première fibre optique (22) pour éclairer la face arrière du micromiroir de palladium (21) en assurant un chauffage localisé de cette face arrière, le coupleur en Y (23) recevant en retour le signal lumineux de longueur d'onde λ réfléchi par le micromiroir de palladium (21) et appliquant ce signal lumineux réfléchi auxdits moyens de détection (25,26).

5. Dispositif selon la revendication 1, **caractérisé en ce qu'**il comprend une première source lumineuse (34) constituée par une diode laser de longueur d'onde λ située dans l'infrarouge et de puissance comprise entre quelques dizaines de milliwatts et quelques centaines de milliwatts pour émettre de façon continue à travers un isolateur optique (35) un signal de détection à ladite deuxième extrémité de la première fibre optique (32) pour éclairer la face arrière du micromiroir de palladium (31) situé à la première extrémité de la première fibre optique (32) en assurant un chauffage localisé de cette face arrière, le signal lumineux de longueur d'onde λ étant transmis à travers le micromiroir de palladium (31) semi-transparent à une première extrémité d'une deuxième fibre optique (33) mise en vis-à-vis de la deuxième extrémité de la première fibre optique (32) portant le micromiroir de palladium (31), la deuxième fibre optique (33) présentant un diamètre de coeur équivalent ou supérieur à celui de la première fibre optique (32) et appliquant par sa deuxième extrémité le signal lumineux transmis à travers le micromiroir de palladium (31) auxdits moyens de détection (36).

6. Dispositif selon la revendication 5, **caractérisé en ce que** la première fibre optique (32) est fixée par collage au voisinage de sa première extrémité à un support (40) transparent et muni d'orifices (41) de communication avec l'environnement à températures cryogéniques et **en ce que** la deuxième fibre optique (33) est également fixée par collage, au voisinage de sa première extrémité, au support (40) de telle manière que la distance entre la première extrémité de la première fibre (32) et la première extrémité de la deuxième fibre (33) soit de l'ordre de quelques dizaines de micromètres.

7. Dispositif selon l'une quelconque des revendications 1 à 6, **caractérisé en ce que** la première fibre optique (12 ; 22 ; 32) présente un diamètre de coeur de l'ordre de 50 micromètres et un diamètre de gaine de l'ordre de 125 micromètres.

8. Dispositif selon l'une quelconque des revendications 1 à 7, **caractérisé en ce que** le micromiroir de palladium (11 ; 22 ; 31) est constitué par un film dont l'épaisseur est comprise entre 10 et 50 nanomètres, et de préférence comprise entre 15 et 30 nanomètres.

9. Dispositif selon l'une quelconque des revendications 1 à 8, caractérisé en que l'environnement à températures cryogéniques est constitué par une atmosphère gazeuse telle que de l'azote.

10. Dispositif selon l'une quelconque des revendications 1 à 8, **caractérisé en ce que** l'environnement à températures cryogéniques est constitué par un fluide liquide tel qu'une huile de refroidissement.

## Claims

1. An optical device for in situ detection of traces of gaseous hydrogen in an environment at cryogenic temperature, using a sensor having a fine film of palladium whose optical properties vary as a function of the concentration of hydrogen in contact with the film, the device comprising a palladium micromirror (11; 21; 31) deposited in the form of a film at a first end of a first optical fiber (12; 22; 32), a first light source (14; 24; 34) emitting a light signal at a predetermined wavelength into a second end of the first optical fiber (12; 22; 32), means (16; 26; 36) for detecting the light signal as modified after passing in contact with the palladium micromirror (11; 21; 31) that is disposed in the environment at cryogenic temperature, processor circuits (17; 27; 37) for responding to variation in the intensity of the light signal that has been in contact with the palladium micromirror (11; 21; 31) to determine the hydrogen concentration, and localized heating means for heating the palladium micromirror (11; 21; 31) by light radiation, so as to maintain the micromirror in the α phase of palladium.

2. A device according to claim 1, **characterized in that** it comprises a first light source (14) constituted by a laser diode of wavelength λ₁ situated in the near infrared and of power that is less than a few tens of milliwatts for emitting a detection signal via a Y coupler (13) into said second end of the first optical fiber (12) so as to illuminate the rear face of the palladium micromirror (11), the Y coupler (13) receiving in return the light signal of wavelength λ₁ reflected by the palladium micromirror (11) and applying said reflected light signal via an interference filter (15) to said detector means (16), and **in that** the means for localized heating of the palladium micromirror (11) by light radiation comprise a second light source (1) constituted by a laser diode of wavelength λ₂ situated in the infrared, longer than the wavelength λ₁ and continuously emitting a beam of power lying in the range a few tens to several hundreds of milliwatts, into a first end of a second optical fiber (2) whose second end is situated at a short distance from the palladium micromirror (11) in the environment at cryogenic temperature so as to heat the front face of the micromirror (11) by the light radiation from the second light source (1) and the optical fiber (2).

3. A device according to claim 2, **characterized in that** the first end of the first optical fiber (12) and the second end of the second optical fiber (2) are united by a ferrule (3) of transparent material provided with orifices (4) for communication with the environment at cryogenic temperature.

4. A device according to claim 1, **characterized in that** it comprises a first light source (24) constituted by a laser diode of wavelength λ situated in the infrared and of power lying in the range a few tens of milliwatts to several hundreds of milliwatts for continuously emitting a detection signal via a Y coupler (23) into said second end of the first optical fiber (22) to illuminate the rear face of the palladium micromirror (21) while simultaneously applying localized heating to said rear face, the Y coupler (23) receiving in return the light signal of wavelength λ as reflected by the palladium micromirror (21) and applying said reflected light signal to said detection means (25, 26).

5. A device according to claim 1, **characterized in that** it comprises a first light source (34) constituted by a laser diode of wavelength λ situated in the infrared and of power lying in the range a few tens of milliwatts to several hundreds of milliwatts for continuously emitting a detection signal via an optical isolator (35) into said second end of the first optical fiber (32) to illuminate the rear face of the palladium micromirror (31) situated at the first end of the first optical fiber (32), thereby providing localized heating of said rear face, the light signal of wavelength λ being transmitted through the semitransparent palladium micromirror (31) to a first end of a second optical fiber (33) placed facing the second end of the first optical fiber (32) carrying the palladium micromirror (31), the second optical fiber (33) having a core of diameter that is equivalent to or greater than the core diameter of the first optical fiber (32), and, at its second end, applying the light signal transmitted through the palladium micromirror (31) to said detection means (36).

6. A device according to claim 5, **characterized in that** adhesive fixes the first optical fiber (32) close to its first end to a transparent support (40) provided with orifices (41) for communicating with the environment at cryogenic temperature, and **in that** adhesive also fixes the second optical fiber (33) close to its first end to the support (40) in such a manner that the distance between the first end of the first fiber (32) and the first end of the second fiber (33) is of the order of a few tens of micrometers.

7. A device according to any one of claims 1 to 6, **characterized in that** the first optical fiber (12; 22; 32) has a core diameter of about 50 micrometers and a cladding diameter of about 125 micrometers.

8. A device according to any one of claims 1 to 7, **characterized in that** the palladium micromirror (11; 22; 31) is constituted by a film of thickness lying in the range 10 nm to 50 nm, and preferably in the range 15 nm to 30 nm.

9. A device according to any one of claims 1 to 8, **characterized in that** the environment at cryogenic temperature is constituted by an atmosphere of gas such as nitrogen.

10. A device according to any one of claims 1 to 8, **characterized in that** the environment at cryogenic temperature is constituted by a liquid such as a cooling oil.

## Patentansprüche

1. Optische Vorrichtung zur in situ-Erfassung von Spuren gasförmigen Wasserstoffs in einer Tiefsttemperatur-Umgebung ausgehend von einem Aufnehmer, der einen dünnen Film aus Palladium aufweist, dessen optische Eigenschaften in Abhängigkeit von der Wasserstoff-Konzentration in Berührung mit dem Film variieren, wobei die Vorrichtung aufweist einen Mikrospiegel aus Palladium (11; 21; 31), der in Film-Form an einem ersten Ende einer ersten optischen Faser (12; 22; 32) abgeschieden ist, eine erste Lichtquelle (14; 24; 34), die ein Lichtsignal vorbestimmter Wellenlänge emittiert, an einem zweiten Ende der ersten optischen Faser (12; 22; 32), Nachweismittel (16; 26; 36) des nach Durchgang unter Berührung des Mikrospiegels aus Palladium (11; 21; 31), der in der Tiefsttemperatur-Umgebung angeordnet ist, modifizierten Lichtsignals, Verarbeitungsschaltungen (17; 27; 37) zur Bestimmung, ausgehend von der Intensitäts-Veränderung des Lichtsignals bei Berührung des Mikrospiegels aus Palladium (11; 21; 31), der Wasserstoff-Konzentration, und Mittel zum örtlich begrenzten Heizen des Mikrospiegels aus Palladium (11; 21; 31) durch Lichtstrahlung, dergestalt, daß der Mikrospiegel in der Alpha-Phase des Palladiums gehalten wird.

2. Vorrichtung nach Anspruch 1,
**dadurch gekennzeichnet, daß**
sie eine erste Lichtquelle (14) aufweist, die gebildet wird von einer Laserdiode einer im nahen Infrarot liegenden Wellenlänge λ₁ und einer unter einigen 10 mW liegenden Leistung, um über einen Y-Koppler (13) zu dem zweiten Ende der ersten optischen Faser (12) ein Nachweissignal zu emittieren, um die Rückseite des Mikrospiegels aus Palladium (11) zu beleuchten, wobei der Y-Koppler (13) wiederum das von dem Mikrospiegel aus Palladium (11) reflektierte Lichtsignal der Wellenlänge λ₁ empfängt und dieses reflektierte Lichtsignal über einen Interferenzfilter (15) an die Nachweismittel (16) übermittelt, und dadurch, daß die Mittel zum örtlich begrenzten Heizen des Mikrospiegels aus Palladium (11) durch Lichtstrahlung eine zweite Lichtquelle (1) aufweisen, die gebildet wird von einer Laserdiode einer im Infraroten liegenden Wellenlänge λ₂, die größer ist als die Wellenlänge λ₁ und die in kontinuierlicher Weise einen Strahl einer Leistung, die zwischen einigen 10 und einigen 100 mW liegt, zu einem ersten Ende einer zweiten optischen Faser (2) emittiert, von der sich ein zweites Ende in geringem Abstand zu dem Mikrospiegel aus Palladium (11) in der Tiefsttemperatur-Umgebung befindet, dergestalt, daß ein Aufheizen der Wand vor dem Mikrospiegel (11) durch die aus der zweiten Lichtquelle (1) und der optischen Faser (2) hervorgegangene Lichtstrahlung sichergestellt wird.

3. Vorrichtung nach Anspruch 2,
**dadurch gekennzeichnet, daß**
das erste Ende der ersten optischen Faser (12) und das zweite Ende der zweiten optischen Faser (2) durch einen Bundring (3) aus transparentem Material, der mit Öffnungen (4) zur Verbindung mit der Tiefsttemperatur-Umgebung versehen ist, zusammengefügt sind.

4. Vorrichtung nach Anspruch 1,
**dadurch gekennzeichnet, daß**
sie eine erste Lichtquelle (24) aufweist, die gebildet wird von einer Laserdiode einer im Infraroten liegenden Wellenlänge λ und einer zwischen einigen 10 mW und einigen 100 mW liegenden Leistung, um in kontinuierlicher Weise über einen Y-Koppler (23) ein Nachweissignal zu dem zweiten Ende der ersten optischen Faser (22) zu emittieren, um die Rückseite des Mikrospiegels aus Palladium (21) zu beleuchten unter Sicherstellung einer örtlichen Erwärmung dieser Rückseite, wobei der Y-Koppler (23) wiederum das von dem Mikrospiegel aus Palladium (21) reflektierte Lichtsignal einer Wellenlänge λ empfängt und dieses reflektierte Lichtsignal an die Nachweismittel (25, 26) übermittelt.

5. Vorrichtung nach Anspruch 1,
**dadurch gekennzeichnet, daß** sie eine erste Lichtquelle (34) aufweist, die gebildet wird von einer Laserdiode einer im Infraroten liegenden Wellenlänge λ und einer zwischen einigen 10 mW und einigen 100 mW liegenden Leistung, um in kontinuierlicher Weise über einen optischen Einwegleiter (35) ein Nachweissignal zu dem zweiten Ende der ersten optischen Faser (32) zu emittieren, um die Rückseite des Mikrospiegels aus Palladium (31), der am ersten Ende der ersten optischen Faser (32) liegt, zu beleuchten unter Sicherstellung einer örtlich begrenzten Erwärmung dieser Rückseite, wobei das Lichtsignal der Wellenlänge λ über den halbdurchlässigen Mikrospiegel aus Palladium (31) an ein erstes Ende einer zweiten optischen Faser (33), das gegenüber dem zweiten Ende der ersten optischen Faser (32), die den Mikrospiegel aus Palladium (31) trägt, angeordnet ist, übertragen wird, wobei die zweite optische Faser (33) einen Kerndurchmesser aufweist, der gleich oder größer ist als derjenige der ersten optischen Faser (32), und über ihr zweites Ende das über den Mikrospiegel aus Palladium (31) übertragene Lichtsignal an die Nachweismittel (36) übermittelt.

6. Vorrichtung nach Anspruch 5,
**dadurch gekennzeichnet, daß**
die erste optische Faser (32) nahe an ihrem ersten Ende durch Klebung an einem transparenten und mit Öffnungen (41) zur Verbindung mit der Tiefsttemperatur-Umgebung versehenen Halter (40) befestigt ist, und dadurch, daß die zweite optische Faser (33) gleichermaßen durch Klebung, nahe an ihrem ersten Ende, an dem Halter (40) befestigt ist, dergestalt, daß der Abstand zwischen dem ersten Ende der ersten Faser (32) und dem ersten Ende der zweiten Faser (33) in der Größenordnung einiger 10 µm ist.

7. Vorrichtung nach einem der Ansprüche 1 bis 6,
**dadurch gekennzeichnet, daß**
die erste optische Faser (12; 22; 32) einen Kerndurchmesser in der Größenordnung von 50 µm und einen Hüllendurchmesser in der Größenordnung von 125 µm aufweist.

8. Vorrichtung nach einem der Ansprüche 1 bis 7,
**dadurch gekennzeichnet, daß**
der Mikrospiegel aus Palladium (11; 21; 31) von einem Film gebildet wird, dessen Dicke zwischen 10 und 50 nm, und bevorzugt zwischen 15 und 30 nm, liegt.

9. Vorrichtung nach einem der Ansprüche 1 bis 8,
**dadurch gekennzeichnet, daß**
die Tiefsttemperatur-Umgebung von einer gasförmigen Atmosphäre wie Stickstoff gebildet wird.

10. Vorrichtung nach einem der Ansprüche 1 bis 8,
**dadurch gekennzeichnet, daß**
die Tiefsttemperatur-Umgebung von einem flüssigen Fluid wie einem Kühlöl gebildet wird.
